(19) Europäisches Patentamt / European Patent Office / Office européen des brevets

(11) **EP 3 893 248 B1**

(12) **EUROPEAN PATENT SPECIFICATION**

(45) Date of publication and mention
of the grant of the patent:
**21.05.2025  Bulletin 2025/21**

(21) Application number: **20461527.2**

(22) Date of filing: **09.04.2020**

(51) International Patent Classification (IPC):
*G16H 50/20* (2018.01)      *G16H 50/70* (2018.01)

(52) Cooperative Patent Classification (CPC):
**G16H 50/20; G16H 50/70**

(54) **METHOD FOR CREATING A PREDICTIVE MODEL FOR PREDICTING GLAUCOMA RISK IN A SUBJECT, METHOD FOR DETERMINING GLAUCOMA RISK IN A SUBJECT USING SUCH PREDICTIVE MODEL, DEVICE FOR PREDICTING GLAUCOMA RISK IN A SUBJECT, COMPUTER PROGRAM AND COMPUTER READABLE MEDIUM**

VERFAHREN ZUR ERSTELLUNG EINES VORHERSAGEMODELLS ZUR VORHERSAGE DES GLAUKOMRISIKOS IN EINEM SUBJEKT, VERFAHREN ZUR BESTIMMUNG DES GLAUKOMRISIKOS IN EINEM SUBJEKT UNTER VERWENDUNG EINES SOLCHEN VORHERSAGEMODELLS, VORRICHTUNG ZUR VORHERSAGE DES GLAUKOMRISIKOS IN EINEM SUBJEKT, COMPUTERPROGRAMM UND COMPUTERLESBARES MEDIUM

PROCÉDÉ DE CRÉATION D'UN MODÈLE PRÉDICTIF POUR PRÉDIRE LE RISQUE DE GLAUCOME CHEZ UN SUJET, PROCÉDÉ DE DÉTERMINATION DU RISQUE DE GLAUCOME CHEZ UN SUJET UTILISANT UN TEL MODÈLE PRÉDICTIF, DISPOSITIF DE PRÉDICTION DU RISQUE DE GLAUCOME CHEZ UN SUJET, PROGRAMME INFORMATIQUE ET SUPPORT LISIBLE PAR ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.10.2021  Bulletin 2021/41**

(73) Proprietors:
• **Instytut Chemii Bioorganicznej PAN**
  **61-704 Poznan (PL)**
• **Wasilewicz, Robert Henryk**
  **61-048 Poznan (PL)**

(72) Inventors:
• **WASILEWICZ, Robert Henryk**
  **61-048 Poznan (PL)**
• **MAZUREK, Cezary**
  **61-139 Poznan (PL)**
• **PUKACKI, Juliusz**
  **61-139 Poznan (PL)**
• **SWIERCZYNSKI, Hubert**
  **61-139 Poznan (PL)**

(74) Representative: **Augustyniak, Magdalena Anna et al**
**Polservice**
**Kancelaria Rzecznikow**
**Patentowych sp. z o.o.**
**Bobrowiecka 8**
**00-728 Warszawa (PL)**

(56) References cited:
• **KIM YOON JEON ET AL: "Ocular pulse amplitude as a dynamic parameter and its relationship with 24-h intraocular pressure and blood pressure in glaucoma", EXPERIMENTAL EYE RESEARCH, ACADEMIC PRESS LTD, LONDON, vol. 115, 21 June 2013 (2013-06-21), pages 65 - 72, XP028733432, ISSN: 0014-4835, DOI: 10.1016/ J.EXER.2013.06.010**
• **MARTIN KEITH R ET AL: "Use of Machine Learning on Contact Lens Sensor-Derived Parameters for the Diagnosis of Primary Open-angle Glaucoma", AMERICAN JOURNAL OF OPHTHALMOLOGY, ELSEVIER, AMSTERDAM, NL, vol. 194, 25 July 2018 (2018-07-25), pages 46 - 53, XP085491561, ISSN: 0002-9394, DOI: 10.1016/ J.AJO.2018.07.005**

EP 3 893 248 B1

- GISLER CHRISTOPHE ET AL: "Towards glaucoma detection using intraocular pressure monitoring", 2014 6TH INTERNATIONAL CONFERENCE OF SOFT COMPUTING AND PATTERN RECOGNITION (SOCPAR), IEEE, 11 August 2014 (2014-08-11), pages 255 - 260, XP032720311, DOI: 10.1109/SOCPAR.2014.7008015
- ROBERT HENRYK WASILEWICZ ET AL: "Daily biorhythms of ocular volume changes and the cardiovascular system functional parameters in healthy, ocular hypertension, normal tension and primary open angle glaucoma populations. | IOVS | ARVO Journals", ARVO ANNUAL MEETING ABSTRACT, 1 April 2014 (2014-04-01), XP055722121, Retrieved from the Internet <URL:https://iovs.arvojournals.org/article.aspx?articleid=2266649&resultClick=1> [retrieved on 20200812]
- R WASILEWICZ ET AL: "24 hour continuous ocular tonography Triggerfish and biorhythms of the cardiovascular system functional parameters in healthy and glaucoma populations - WASILEWICZ - 2013 - Acta Ophthalmologica - Wiley Online Library", 6 August 2013 (2013-08-06), XP055722164, Retrieved from the Internet <URL:https://onlinelibrary.wiley.com/doi/abs/10.1111/j.1755-3768.2013.2721.x> [retrieved on 20200812]
- CAROLINA N. SUSANNA ET AL: "A Prospective Longitudinal Study to Investigate Corneal Hysteresis as a Risk Factor for Predicting Development of Glaucoma", AMERICAN JOURNAL OF OPHTHALMOLOGY, vol. 187, 1 March 2018 (2018-03-01), AMSTERDAM, NL, pages 148 - 152, XP055722492, ISSN: 0002-9394, DOI: 10.1016/j.ajo.2017.12.018
- AZADEH DOOZANDEH ET AL: "Corneal profile in primary congenital glaucoma", ACTA OPHTHALMOLOGICA: THE OPHTHALMOLOGICAL JOURNAL OF THE NORDIC COUNTRIES, vol. 95, no. 7, 31 January 2017 (2017-01-31), Denmark, pages e575 - e581, XP055722493, ISSN: 1755-375X, DOI: 10.1111/aos.13357

## Description

## FIELD

The present invention relates to a method for creating a predictive model for predicting glaucoma risk in a subject, a method for determining glaucoma risk in a subject using such predictive model, a device for predicting glaucoma risk in a subject for performing said methods, a computer program and a computer readable medium. In particular, the invention relates to creating and using the predictive model for predicting glaucoma in a subject which is based on specific features determined from an eyeball biorhythm, using artificial intelligence and machine learning methods.

## BACKGROUND OF THE INVENTION

Glaucoma is a progressive optic neuropathy which is the most common cause of irreversible vision loss in the world. Its pathomechanism is associated with a change in lamina cribrosa phenotype under the influence of excessive mechanical forces, which leads to neurotrophic deprivation and subsequent accelerated process of retinal ganglion cell apoptosis. Loss of retinal ganglion cells leads to disruption of the functional visual pathway continuity which leads to the development of specific, depending on the architecture of the retina and optic nerve defects in the visual field. Those defects appear clinically when at least 30 to 50% of the ganglion cells of a given area of the retina is lost.

Elevated intraocular pressure is the most important known risk factor for the development of glaucomatous optic neuropathy (specifity 0.85 / sensitivity 0.3 / AUC 0.6), and its reduction is the only clinically proven way to reduce the risk of its progression. Modern practice in the field of glaucoma diagnosis or its risk assessment which is based mainly or exclusively on the assessment of intraocular pressure elevation in extreme cases may result in erroneous diagnosis and serious consequences for the subject, especially in cases of glaucoma presence despite of normal intraocular pressure. Furthermore, elevated intraocular pressure may have various grounds which further impairs a correct diagnosis. In order to provide a more effective diagnosis, in addition to the intraocular pressure test, other methods may also be used to assess the risk of glaucoma, such as, for example, fundus examination, visual field examination or optical tomography. However, performing a number of tests is uncomfortable for the subject, time-consuming, and the final assessment of the subject's health state requires many factors to be taken into consideration, and therefore is complicated and prone to errors.

In view of the above, there is a need in the prior art to develop a more effective and reliable method for determining glaucoma risk that will allow to simplify the subject examination process and will provide a quick and reliable diagnosis.

Therefore, it is the object of the present invention to provide a suitable method and a device for predicting glaucoma risk in a subject which at least mitigate the above-mentioned one or more problems in the prior art.

Document Wasilewicz RH, Wasilewicz PK, Mazurek C, et al. "Daily biorhythms of ocular volume changes and the cardiovascular system functional parameters in healthy, ocular hypertension, normal tension and primary open angle glaucoma populations", ARVO Annual Meeting Abstract, April 2014, describes relationships between eyeball biorhythm parameters and cardiovascular system parameters in individual ranges of the 24-hour period.

Document Martin Keith R et al: "Use of Machine Learning on Contact Lens Sensor-Derived Parameters for the Diagnosis of Primary Open-angle Glaucoma", American Journal of Ophthalmology, Elsevier, Amsterdam, NL, vol. 194, 25 July 2018 (2018-07-25), pages 46-53, XP085491561, ISNN: 0002-9394, DOI: 10.1016/J.AJO.2018.07.005, refers to a development and evaluation of a diagnostic test with machine learning to verify that contact lens sensor (CLS)-based 24-hour profiles of ocular volume changes contain information complementary to intraocular pressure (IOP) to discriminate between primary open-angle glaucoma (POAG) and healthy (H) eyes.

Document Gisler Christophe et al: "Towards glaucoma detection using intraocular pressure monitoring", 2014 6th International Conference of Soft Computing and Pattern Recognition (SoCPaR), IEEE 11 August 2014 (2014-08-11), pages 255-260, XP032720311, DOI: 10.1109/SOCPAR.2014.7008015, presents the methodologies, signal processing and machine learning algorithms elaborated in the task of automated detection of glaucomatous IOP-related profiles within a set of 100 24-hour recordings.

The starting point for the development of the present invention was the development of a method for determining characteristic features in the eyeball biorhythm parameters by the inventors, which features proved to be helpful in predicting glaucoma. Determined features enabled a predictive model to be created which on the basis of the above-mentioned features determined for a sufficiently large group of examined and diagnosed subjects is able to classify an examined subject as healthy/diseased with determined probability based on an analogous feature set. Such indication based on previous diagnoses for subjects for which identical features have been determined may be very helpful for the initial assessment of glaucoma risk and can assist physicians in making a diagnosis and proposing a better treatment method. Furthermore, the developed method may be based on an extended feature set including, among others, eyeball parameter and cardiovascular system parameter correlations, subject's age, corneal resistance factor and corneal hysteresis.

## SUMMARY OF INVENTION

According to the invention a method for creating a predictive model for predicting glaucoma risk in a subject according to claim 1, a method for determining glaucoma risk in a subject using the predictive model according to claim 11, a device for predicting glaucoma risk in a subject according to claim 12, and a computer program and a computer readable medium according to claims 14 and 15 respectively are provided.

The method for creating a predictive model for predicting glaucoma risk in a subject according to the invention includes a step of creating a diagnostic model comprising, for each one of a plurality of subjects:

a) recording a 24-hour profile of eyeball parameters and simultaneously recording cardiovascular system parameters;

b) dividing the recorded 24-hour profile of eyeball parameters at least into subperiods: an initial subperiod from session start to 5 hours before assuming a horizontal position for sleep; a subperiod preceding assuming a horizontal position for sleep from 5 hours before assuming a horizontal position to assuming a horizontal position for sleep; a subperiod following assuming a horizontal position for sleep from assuming a horizontal position for sleep to assuming a horizontal position for sleep + 2 hours; a subperiod preceding assuming a vertical position after sleep from assuming a horizontal position for sleep + 2 hours to assuming a vertical position after sleep; a subperiod following assuming a vertical position after sleep from assuming a vertical position after sleep to assuming a vertical position after sleep + 2 hours; a final subperiod from assuming a vertical position after sleep + 2 hours to the session end;

c) determining, in each subperiod, features describing a single subject in the form of at least one aggregating attribute and calculating correlations between the eyeball parameters and the cardiovascular system parameters;

d) creating a record containing the determined features describing a single subject and the calculated correlation parameters;

e) assigning a label indicating a diagnosis (diseased/healthy) made by a physician to the created record.

Furthermore, said method according to the invention includes a step of creating a predictive model, based on a set of records created for the plurality of subjects, using supervised machine learning mechanisms based on one or more algorithms selected at least from regression algorithms, decision trees, Bayesian algorithms, ensemble algorithms and support vector-based algorithms. The predictive model receives a record of a patient to be examined, the record containing the same feature set as the one created in step d) and outputs the probability of said record to belong to a group of healthy subjects or to a group of diseased subjects.

In a preferred embodiment of the method, the predictive model is created using 10-fold cross-validation.

In another preferred embodiment of the method, the aggregating attributes are selected from a group including: a sum of the area under the curve in a subperiod, the slope angle of a linear regression line in a subperiod, the total variation in a subperiod, representative values of the discrete Fourier transform in a subperiod.

In yet another preferred embodiment of the method, the eyeball parameters are selected from a group including: the circumference at the corneoscleral limbus of an eyeball and intraocular pressure.

In yet another preferred embodiment of the method, the cardiovascular system parameters are selected from a group including: blood pressure (BP): systolic arterial pressure (SAP), diastolic arterial pressure (DAP), mean arterial pressure (MAP), heart rate (HR), oxygen blood saturation (SpO2) and cardiac output fraction calculated according to the formula: CO = [(SAP-DAP)/SAP + DAP)] x HR.

In yet another preferred embodiment of the method, one or more additional features selected from a group including: subject's age, corneal resistance factor and corneal hysteresis are determined and appended to the record describing a single subject created in the step of creating a record.

In yet another preferred embodiment of the method, the record describing a single subject is limited to a selected subset of the all determined features.

In yet another preferred embodiment of the method, the determined subperiods furthermore include a subperiod from the session start to assuming a horizontal position for sleep and/or a subperiod from assuming a horizontal position for sleep to assuming a vertical position after sleep and/or a subperiod from assuming a horizontal position at 14:00 to assuming a vertical position at 15:30 with sustained consciousness.

A method for determining glaucoma risk in a subject according to the invention includes: creating, for a patient to be examined, a record containing the same feature set as the one created in the step of creating a record of the method for creating a predictive model disclosed above, and determining the subject' feature similarity to a group of diseased or healthy subjects with determined probability using the predictive model created according to the method for creating a predictive model disclosed above.

A device for predicting glaucoma in a subject according to the invention comprises means for recording eyeball parameters; means for recording cardiovascular system parameters; a control circuit having a communication connection with both the means for recording, and comprising: a processor; a memory operatively coupled to the processor, containing instructions that, when executed by the processor, implement the method as defined above based on data provided by the means for recording; and an output device for presenting results having a communication connection with the control circuit.

In a preferred embodiment of the device, the means for recording eyeball parameters, the means for recording cardiovascular system parameters and/or the output device are arranged in a remote location with respect to the control circuit, and the communication connections are communication network connections.

The invention also relates to a computer program comprising a program code that, when executed by a processor, causes the processor to perform the method steps according to the invention and to a computer readable medium on which the computer program is stored.

The solutions provided according to the invention find application in an intelligent physician decision support system which enables an ultra-early risk assessment of glaucoma neuropathy and the description of its factors in the population of people observed for glaucoma, subjects with a positive family history and ocular hypertension. As a result it is possible to personalize the methods of local and systemic therapy in glaucomatous subjects with an indication of individual risk factors for disease progression.

Further features and advantages of the present invention will become apparent after reading the description presented below in connection with the attached drawing, in which

Fig. 1    shows a schematic block diagram illustrating successive steps of a method for creating a predictive model according to the invention.

Fig. 2    shows an exemplary 24-hour variation profile of an eyeball circumference at the corneoscleral limbus.

Fig. 3    shows an exemplary 24-hour variation profile of intraocular pressure.

Fig. 4    shows an exemplary 24-hour variation profile of functional cardiovascular system parameters.

Fig. 5    shows an exemplary 24-hour variation profile of an eyeball circumference at the corneoscleral limbus.

Fig. 6    shows a signal amplitude/pressure relationship as a function of time using the ocular response analyser.

Fig. 7    shows a schematic block diagram of an exemplary device for predicting glaucoma risk in a subject.

## DETAILED DESCRIPTION OF EMBODIMENTS OF THE INVENTION

Preferred embodiments are described below. Details of particular embodiments of the method apply at the same time, in the relevant scope, to embodiments of the device. Therefore, the repeated description will be omitted.

The subject solution is mainly based on the processing and analysis of data from daily eyeball biorhythm measurements and, optionally, from daily cardiovascular system parameter measurements. Additionally, permanent patient characteristics such as age, corneal resistance factor or corneal hysteresis can be used as a variant, which will be described in more detail below.

In order to perform the analytical process, it is necessary to construct a predictive model that will be used to determine a predicted health state of a subject along with probability of assessment accuracy. In practice, this will be an indication for the physician that a person classified as diseased has parameters similar to those of people diagnosed with the disease. It will not necessarily be an indication that the disease has already occurred.

In order to perform an analysis for a given subject, it is necessary to provide raw data from eyeball biorhythm monitoring devices and, optionally, changes in cardiovascular parameters over a 24-hour profile. The presented embodiment is based on data from specific devices described below.

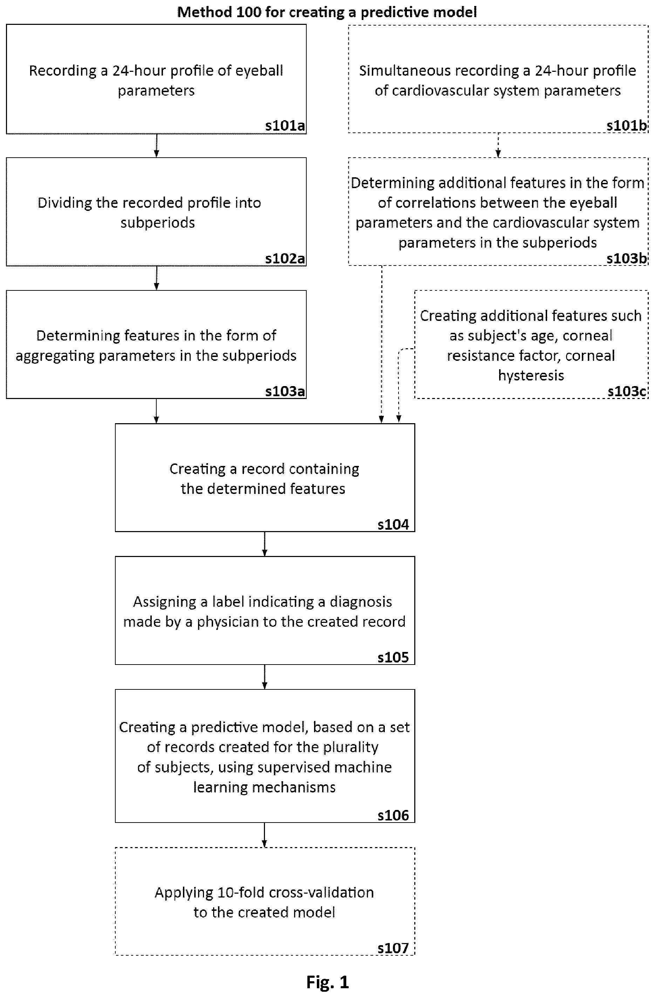

Fig. 1 shows a schematic block diagram illustrating successive steps of the method **100** for creating a predictive model for determining predicted health state of a subject. Blocks and arrows describing the relationships between blocks in Fig. 1 shown using dashed lines present a preferred addition to the described method **100,** but are not necessary for its functioning.

In order to create a predictive model it is necessary to gather a sufficiently large set of data describing a diverse group of subjects. Successive steps **s101a, s102a, s103a, s104** and **s105** of the method **100** described below, similarly as optional steps **s101b, s103b** and **s103c,** are therefore analogously performed for each one of a plurality of subjects in order to provide so-called training data.

In step **s101a** a 24-hour profile of eyeball parameters is recoded. At this point it is noted that as eyeball parameters all parameters should be understood which describe features of an eyeball, such as, e.g., the circumference at the corneoscleral limbus of an eyeball, intraocular pressure (IOP) and similar parameters describing the variation of an eyeball biorhythm but these are only non-limiting examples. The person skilled in the art will notice other relevant eyeball parameters that are not specifically mentioned here. All the parameters listed above are values which are variable both individually and as a function of time. Data sequences resulting from the registration of the above parameters as a function of time for the 24-hour period are also referred to as the biorhythms in the present disclosure.

In optional step **s101b,** simultaneously with step **s101a**, a 24-hour profile of cardiovascular system parameters is recorded. At this point it is noted that as cardiovascular system parameters all parameters should be understood which describe cardiovascular system, such as, e.g., blood pressure (BP): systolic arterial pressure (SAP), diastolic arterial pressure (DAP), mean arterial pressure (MAP), heart rate (HR), oxygen blood saturation (SpO2) and cardiac output fraction calculated according to the formula: CO = [(SAP-DAP)/SAP + DAP)] x HR, but these are only non-limiting examples. The person skilled in the art will notice other relevant cardiovascular system parameters that are not specifically mentioned here. All the parameters listed above are values which are variable both individually and as a function of time. Data sequences resulting from the registration of the above parameters as a function of time for the 24-hour period are also referred to as the biorhythms in the present disclosure.

For the recording of the above-mentioned biorhythms, any commercially available systems can be used, such as e.g. Triggerfish, PMCL, Somnotouch NIBP systems and the like, however the invention is not limited to them and any other devices and systems developed in the future and/or having a similar function can be used as long as they can provide the needed data sequences, both at intervals and in continuous mode.

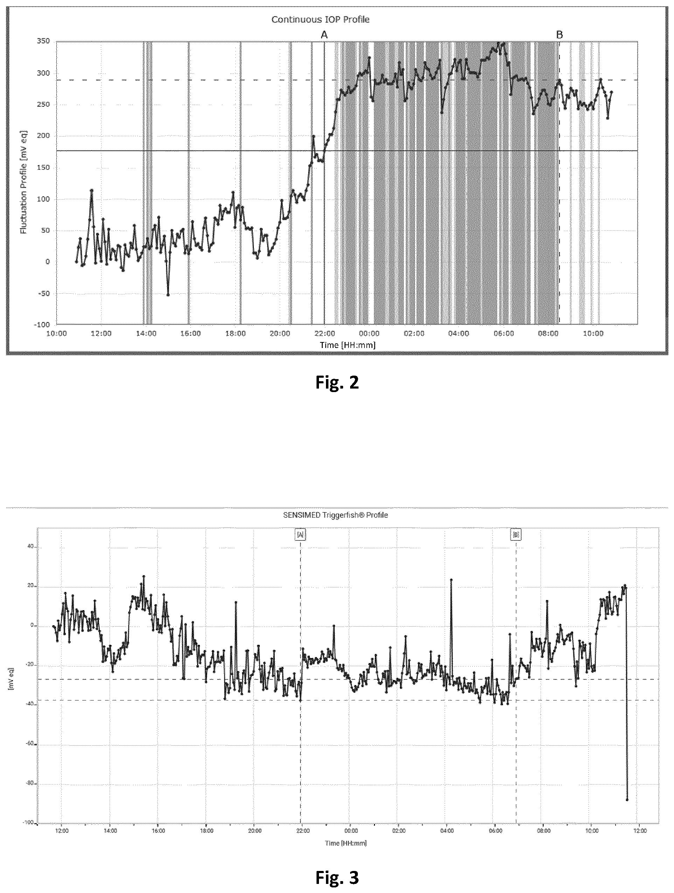

Figures 2-4 show exemplary diagrams of selected parameters as a function of time that can be used in the present invention and can be helpful for understanding the principles of the present invention.

Fig. 2 shows an exemplary 24-hour variation profile of the circumference at the corneoscleral limbus of an eyeball obtained by means of the Triggerfish system (Sensimed AG, Switzerland) which may be used in the present invention. In this system, data is recorded with a frequency of 10 Hz every 5 minutes in a 30-second measurement window. The data unit is mV. The median value of the data sequence from a given measurement window creates a point on the 24-hour timeline. Recording a set of these points at 5-minute intervals creates a time curve describing an individual biorhythm of circumferential dimensional changes at the corneoscleral limbus of an eyeball.

Fig. 3 shows an exemplary 24-hour variation profile of intraocular pressure (IOP) obtained by means of the PMCL system (Sensimed AG, Switzerland) which may be used in the present invention. In this case, the data recording is continuous with division into 180-second measurement windows in which data is recorded within two subperiods; at a frequency of 50 Hz for the first 30 seconds and at a frequency of 1 Hz during the remaining 150 seconds of the measurement window. The data unit is mmHg. The median value of the data sequence from a given measurement window creates a point on the 24-hour timeline. A record of a set of these points at 3-minute intervals creates a time curve describing an individual biorhythm of intraocular pressure variations.

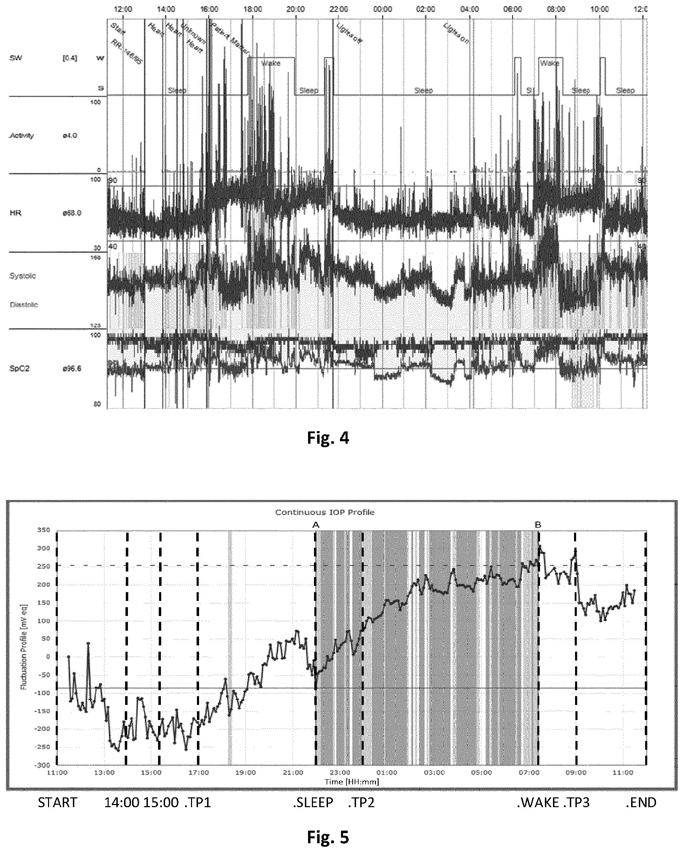

Fig. 4 shows an exemplary 24-hour variation profile of functional cardiovascular system parameters obtained by means of the Somnotouch NIBP system (Somnomedics AG, Germany). This system enables obtaining a 24-hour variation profile of functional cardiovascular system parameters in a continuous mode (beat-to-beat) using the PTT (pulse-transit-time) method. The functional parameters described are: systolic arterial pressure (SAP), diastolic arterial pressure (DAP), mean arterial pressure (MAP), heart rate (HR) and oxygen blood saturation (SpO2) and additionally cardiac output (CO) fraction calculated according to the formula: CO = [(SAP-DAP) / SAP + DAP] x HR. Data units are, respectively: mmHg, number of heart beats per minute, %.

Returning to Fig. 1, the data sequences for the parameters obtained in steps **s101a** and **s101b** are then subjected to a pre-processing to give them the desired form adapted for further processing. In the case of simultaneous recording eyeball parameters and cardiovascular system parameters, it may be necessary to synchronize the recorded data and to provide a compatible form for both sequences. This is especially important in case of data originating from different systems, i.e. recorded in different modes (e.g. continuous/discrete) and/or recorded in a different way e.g. at different frequencies or at different time points. As a result, sets of parameters that correspond to each other in time are obtained on one timeline, which parameters describe the biorhythm of the eyeball on one side and the cardiovascular system on the other. Such arrangement of data enables, among others, correlation calculation for data from both profiles at defined time points, which correlation is to be used in the later stages. In the presented embodiment, the recorded data is subjected to preliminary processing in such a way that parameter values are obtained at time points being full minutes, so that the correlated parameters are the median values of parameters read from devices in a given minute. However, the invention is not limited to this embodiment and any suitable time points can be selected, e.g. with a 30-second, 2-minute, 5-minute increment, etc. Any known techniques may be used to determine individual parameter values at the required time points that take into account adjacent and/or near values, e.g. median calculation, approximation, interpolation, etc.

In step **s102a**, the pre-processed eyeball parameters are allocated to characteristic subperiods where it will be possible to determine the features used to construct the predictive model. In the case where cardiovascular system parameters in the step **s101b** are also recorded, both synchronized profiles from steps **s101a** and **s101b** are divided into said identical subperiods. Fig. 5 shows an exemplary division into subperiods for a 24-hour description of variability of an eyeball circumference at the corneoscleral limbus, wherein identical division also occurs in an analogous way for the cardiovascular system parameters (not shown).

In the presented embodiment, 9 original subperiods of comparative data analysis were determined based on the comparative analysis of biorhythms of circumferential dimensional changes at the corneoscleral limbus of an eyeball, intraocular pressure and ocular pulse amplitude, and the corresponding specificity of human behaviour associated with circadian rhythm. The individual subperiods defined by particular boundary time points **START, TP1, SLEEP, TP2, WAKE, TP3, END** are briefly characterized below:

- From "session start" to "5 hours before assuming a horizontal position for sleep": **START - TP1**
  This subperiod describes relationships between the systems during daily activity (vertical position) of a given person in time when specific volume / IOP change patterns of the eyeball occur, caused mainly by cardiovascular system parameters variability resulting from individual behaviour types specific for a given person.

- From "5 hours before assuming a horizontal position for sleep" to "assuming a horizontal position for sleep": **TP1-SLEEP**
  This subperiod describes relationships between the systems during daily activity of a given person (vertical position), in time when specific changes in the human endocrine system occur that influence the eyeball volume / IOP and cardiovascular system

- From "assuming a horizontal position for sleep" to "assuming a horizontal position for sleep + 2 hours": **SLEEP - TP2**
  This subperiod describes relationships between the systems during the first hours of sleep (horizontal position) of a given person in time when dynamic continuous volume / IOP changes of the eyeball and changes in cardiovascular system parameters occur resulting from the change in body position from vertical to horizontal, sleep and accompanying changes in the human endocrine system.

- From "assuming a horizontal position for sleep + 2 hours" to "assuming a vertical position after sleep": **TP2 - WAKE**
  This subperiod describes relationships between the systems during sleep (horizontal position) of a given person in which time the eyeball volume / IOP change patterns are associated with fixed horizontal body position and associated with it changes in cardiovascular system parameters, blood saturation disorders, and sleep apnea.

- From "assuming a vertical position after sleep" to , assuming a vertical position after sleep + 2 hours": **WAKE - TP3**
  This subperiod describes relationships between the systems during the first hours of daily activity (vertical position) of a given person in time when there are dynamic continuous changes in the eyeball volume and cardiovascular system parameters, resulting from the change in body position from horizontal to vertical, from changes in the human endocrine system and from pathognomonic behaviour of episcleral vessels of the eyeball resulting from their interaction with the sensor of the eyeball volume / IOP change assessment systems in the form of contact lens.

- From "assuming a vertical position after sleep + 2 hours" to the session end: **TP3- END**
  This subperiod describes relationships between the systems during daily activity (vertical position) of a given person in time when specific volume / IOP change patterns of the eyeball occur, caused mainly by the cardiovascular system parameters variability resulting from individual behaviour types specific for a given person.

- From "session start" to "assuming a horizontal position for sleep": **START - SLEEP**
  This subperiod describes relationships between the systems during daily activity of a given person.

- From "assuming a horizontal position for sleep" to "assuming a vertical position after sleep": **SLEEP -WAKE**
  This subperiod describes relationships between the systems during sleep of a given person.

- From "assuming a horizontal position at 14:00" to " assuming a vertical position at 15:30" with sustained consciousness (lying without sleep): **TIME 14:00 - TIME 15:30**
  This subperiod describes relationships between the systems during daily activity of a given person after assuming a horizontal position- functional test.

Although in the presented embodiment 9 subperiods are used which turned out to be beneficial for the implementation of the invention, the skilled person in the art will notice that the presented division is only exemplary and more or less subperiods can be used, and other time points can be selected in a 24-hour profile which will define the boundaries of particular subperiods that are specific for given patient conditions, without departing from the scope of the invention.

In step **s103a,** for each one of a plurality of subjects, based on the processed data of the eyeball parameter profile and within the determined subperiods, features in the form of aggregation parameters are determined which are used to create a diagnostic model. As mentioned earlier, for this purpose a sufficiently large set of data describing a diverse group of subjects should be gathered and used.

The process of determining (extracting) features consists in determining a limited set of features that can be used to construct a diagnostic model using machine learning methods. This is related to the specificity of the machine learning technology which allows to create effective models assuming that the number of features is much smaller than the number of cases based on which the model is created. The median calculation described above also reduces the dimensionality of the feature space but determining aggregation parameters in the context of determined subperiods is of primary importance. Aggregation parameters or aggregation attributes used in the present disclosure should be understood as parameters determined on the basis of measurement values collected (aggregated) in a given subperiod. In the described embodiment, the above process was carried out in full consultation with a field expert who, based on the knowledge of physiological processes described by the data, was able to point to the characteristic elements in the daily profile of the parameter value of the eyeball biorhythm (hereinafter also referred to as TF). As a result of these actions the features described below were determined which in the described embodiment of the invention were subsequently used to create statistical models minimizing the classification error gradient by means of boosting and logistic regression models:

a) Sum of the <u>area under the TF curve in a subperiod</u>

This sum is calculated assuming a constant TF level between successive recorded values/measurements (step curve) in a subperiod. For the selected (e.g. TP1-SLEEP) subperiod $T = \{t_0, t_1,...,t_n\}$ TF measurements $\{tf_0, tf_1,...,tf_n\}$ are stored. The sum of the area under the TF curve is equal to the sum of rectangles for individual measurements, i.e.

$$S_T = \sum_{i=0}^{i=n-1} s_i,$$

where $s_i = (t_{i+1} - t_i) \cdot tf_i$

b) <u>Slope angle of a linear regression line in a subperiod</u>

The slope angle of a linear regression line for TF measurements in a subperiod is calculated by the method of least squares. Initial time $t_0 = 0$ is assumed for each one of the subperiod. The original directional coefficient of the line or the angle value in radians is recorded. The least squares method determines coefficients $\beta = (\beta_0, \beta_1)$ of the line equation on the plane according to formula $\beta = (X^T X)^{-1} X^T y$, for the input data matrix X written with 1 at the first position.

c) <u>Total variation in a subperiod</u>

The total variation in a subperiod is calculated in the form of the numerical integral of the second derivative. For example, the total variation of TF over time period T can be calculated from the formula:

$$V_T = \sum_{i=0}^{i=n-1} |tf_{i+1} - tf_i|$$

d) <u>Representative values of the discrete Fourier transform (FFT)</u>

The discrete Fourier transform is calculated by the FFT method based on the original data from which the linear trend has been removed. Representative FFT values are determined using cluster analysis that uses the DTW (Dynamic Time Warping) metric:

    i. power of the first FFT local maximum taking into account main components (Power_1)

    ii. frequency of the first FFT local maximum taking into account main components (Hz_1)

For the DFT calculation in the present embodiment of the invention, the fft method from stats v3.6.2 package in R was used: (https://www.rdocumentation.org/packages/stats/versions/3.6.2/topics/fft).

String $\{X_k\} = X_0, X_1,...,X_{N-1}$ of the DFT values is calculated for input string $\{x_n\} = x_0, x_1,...,x_{N-1}$ according to the formula:

$$X_k = \sum_{n=0}^{N-1} x_n \cdot e^{-\frac{i2\pi}{N}kn},$$

where k = 0, 1, ..., N - 1

Due to disturbances in the exact profile of the eyeball biorhythm during the day associated with the subject's blinking, the described FFT parameters are preferably calculated only for subperiod TP2-WAKE (sleep).

e) Correlations between eyeball biorhythm values and cardiovascular parameter values in the determined subperiods

In the present embodiment of the invention, correlations between median values of TF parameters and medians of individual cardiovascular system parameters (SAP, DAP, MAP, HR, SPO2, CO) were determined within each subperiod. In general, the correlation calculation can be performed using generally known and available statistical models. In the present embodiment, the correlation calculation was carried out using methods from the psych v1.8 package for the R language.
The Spearman's rank correlation coefficients were calculated using the corr.test method assuming a standard threshold of 0.05 for (alpha) parameter defining the range of confidence intervals. The application of this method minimizes the impact of separated values / measurements (outliers) on the determined correlation coefficients for the analysed parameters. The determined coefficients belong to interval [-1.1] whose endpoints correspond to the total negative and total positive correlation.

The method of calculating correlations is described in more detail below. For n measurements, the original vectors $X, Y$ of length n are converted into rank vectors $X^{rg}, Y^{rg}$. In the simplest case where there are no identical measurements, it can be assumed that rank vectors are indexes of ordered / sorted original vectors. In the case of repetitions of certain values, a procedure assigning rational ranks is used which resolves the ambiguity of the ranking.

$$rho = Cov(X^{rg}, Y^{rg})/(\sigma(X^{rg})\cdot\sigma(Y^{rg})),$$

where rho - Spearman's correlation coefficient, Cov - rank vector covariance, $\sigma$ - standard deviation of the rank vector.
rho coefficient is equal to $\pm 1$ when the relationship between $X, Y$ is a monotonic function. In the case of Pearson's correlation, the corresponding coefficient is equal to $\pm 1$ when the relationship between $X, Y$ is a linear function. The correlation sign specifies the type of relationship between $X, Y$-it is positive for an increasing function and negative for a decreasing function.
The process ends with returning a result in the numerical form and possibly in the graphic form, such as a heat map. When generating a heat map, similar correlation results are additionally grouped (clustering) to visually gather subjects into groups with similar characteristics. The heat map may be helpful for the physician when making a diagnosis, but it is not required for the functioning of solutions according to the invention. Below a table showing the numerical values of correlation between the cardiovascular system parameters (median_DAP, median_HR, median_SAP, median_MAP, median_SpO2, median_CO) and the eyeball biorhythm (TF) parameters for SLEEP-WAKE subperiod is presented.

Table 1. Numerical values of correlation between the cardiovascular system parameters and the eyeball biorhythm parameters for SLEEP-WAKE subperiod

| ID | median_DAP | median_HR | median_SAP | median_MAP | median_SpO2 | median_CO |
|---|---|---|---|---|---|---|
| tf_001 | 0 | 0,41 | 0,3 | 0 | 0 | 0,48 |
| tf_002 | -0,73 | -0,51 | -0,8 | -0,77 | 0 | -0,72 |
| tf_003 | -0,4 | -0,55 | -0,55 | -0,54 | 0 | -0,62 |
| tf_004 | -0,37 | 0 | -0,34 | -0,39 | 0 | 0 |
| tf_005 | -0,65 | 0 | -0,48 | -0,59 | 0 | 0 |
| tf_006 | -0,69 | -0,67 | -0,56 | -0,69 | 0 | -0,57 |
| tf_007 | -0,61 | 0 | -0,82 | -0,71 | 0 | -0,42 |
| tf_008 | -0,73 | -0,5 | -0,66 | -0,71 | -0,35 | -0,5 |
| tf_009 | -0,29 | -0,82 | 0 | 0 | 0 | -0,76 |
| tf_010 | -0,6 | 0,34 | -0,56 | -0,6 | 0 | 0,44 |
| tf_011 | 0 | -0,3 | 0 | 0 | 0 | 0 |

(continued)

| ID | median_DAP | median_HR | median_SAP | median_MAP | median_SpO2 | median_CO |
|---|---|---|---|---|---|---|
| tf_012 | 0 | 0 | 0 | 0 | 0 | 0 |
| tf_013 | -0,37 | 0 | -0,33 | -0,35 | 0 | 0 |
| tf_014 | 0 | 0 | 0 | 0 | 0 | 0 |
| tf_015 | 0 | 0 | -0,33 | -0,32 | 0,39 | 0 |
| tf_016 | -0,29 | -0,45 | -0,37 | -0,34 | 0 | -0,44 |
| tf_017 | 0 | 0 | 0 | 0 | 0 | 0 |
| tf_018 | -0,71 | -0,44 | -0,75 | -0,77 | 0 | -0,49 |
| tf_019 | -0,33 | -0,58 | 0 | 0 | 0 | -0,39 |
| tf_020 | -0,37 | -0,31 | -0,28 | -0,33 | 0 | 0 |
| tf_021 | -0,69 | 0 | -0,67 | -0,68 | 0 | 0 |
| tf_022 | -0,45 | -0,26 | -0,53 | -0,51 | -0,56 | -0,46 |
| tf_023 | -0,59 | -0,3 | -0,47 | -0,55 | -0,4 | -0,33 |
| tf_024 | -0,48 | -0,31 | -0,48 | -0,48 | 0,3 | -0,33 |
| tf_025 | 0,64 | 0,58 | 0,52 | 0,6 | 0,43 | 0,4 |
| tf_026 | -0,69 | 0,72 | 0 | -0,61 | -0,57 | 0,82 |
| tf_027 | -0,37 | 0 | -0,32 | -0,36 | 0 | 0 |
| tf_028 | 0 | 0 | 0 | 0 | 0 | 0 |
| tf_029 | -0,39 | 0 | -0,28 | -0,33 | 0,31 | 0 |
| tf_030 | -0,73 | -0,72 | -0,79 | -0,76 | 0 | -0,65 |
| tf_031 | -0,65 | -0,37 | -0,61 | -0,65 | 0 | -0,34 |

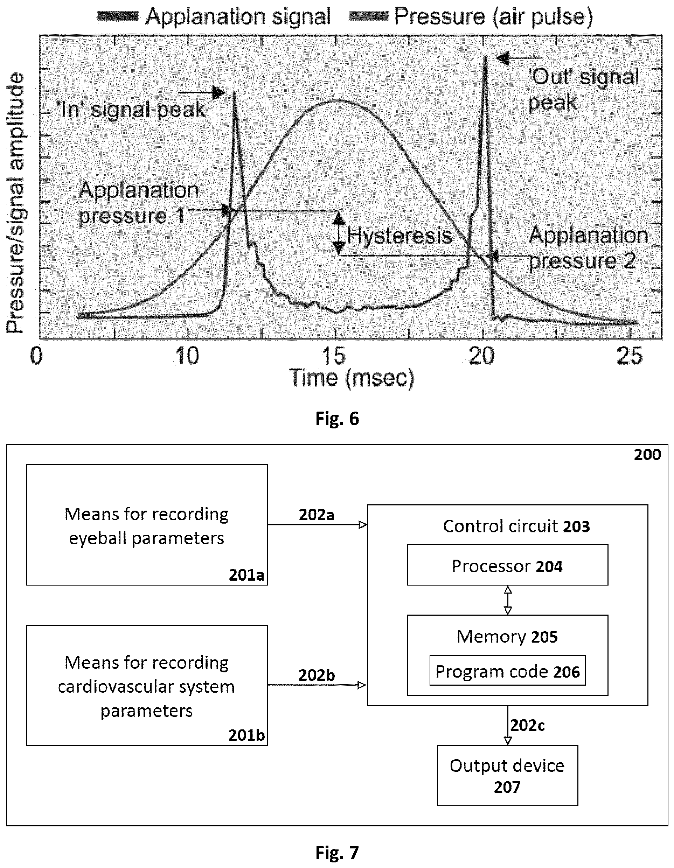

As mentioned at the beginning, when creating a predictive model, in step **s103c** additional parameters can be optionally considered such as subject's age, corneal hysteresis and corneal resistance factor described below with reference to Fig. 6 which shows a signal amplitude/pressure relationship as a function of time using the ocular response analyser.

a) Subject's age - a parameter specifying subject's age at the time of diagnosing.

b) Corneal hysteresis (CH) is an in vivo measurement of cornea biomechanical properties and an independent risk factor for the development and progression of glaucoma neuropathy. Its value reflects the capacity of the corneal tissue to dissipate energy. Corneal hysteresis determines whether the eyeball wall absorbs energy associated with intraocular pressure fluctuations, increasing the risk of lamina cribosa sclerae reconstruction and damage to the optic nerve, or it is able to dissipate this force, protecting the above eye structures from excessive biomechanical loads. Simply put, CH reflects the ability to cushion of the eyeball wall. Eyes that are good shock absorbers (high CH) are less likely to develop glaucoma neuropathy and less often experience its progression. Conversely, poorly cushioning (low CH) eyes are more likely to develop glaucoma and glaucoma is more likely to progress. Average CH for the population for most ethnic groups is around 10 mmHg.
Corneal hysteresis is a numerical value that is automatically generated when performing a measurement by the ORA (Ocular Response Analyser) from Reichert. This device functions very much like a noncontact tonometer. A metered puff of air is delivered to the cornea, flattening it into an applanation configuration (maximum flattening). Then the air puff continues to deform the cornea past this point resulting in a transitory concave corneal configuration. As the pressure of the air puff diminishes, the cornea returns to its normal configuration, passing from the concave configuration, through the applanation position (maximum flattening) to the convex configuration. The pressure of the air puff at the point of the first and second applanations is different (being lower during the second), as the cornea's viscoelastic nature dissipates some of the energy. The difference in IOP at each of these two applanation points is defined as the corneal hysteresis. If the cornea were perfectly elastic and did not dampen some of the energy, the two applanation points would occur at the same IOP level.

c) Corneal resistance factor (CRF)

The CRF is derived from the formula (P1-kP2) where k is the constant determined from an empirical analysis of the relationship between P1 (first applanation pressure), P2 (second applanation pressure) and CCT (central corneal thickness). The CRF describes corneal resistance - its elastic properties. CRF is believed to represent corneal elasticity, with stronger correlations with IOP and CCT compared to CH.

Based on a set of data concerning subjects who were diagnosed, a predictive model is created. For this purpose a record is created in step **s104** for each of the diagnosed patients, the record containing the features determined in step **s103a** as well as optionally the features determined in steps **s103d** and/or **s103c.**

Where appropriate, a specific subset can be selected from all the determined features to be used in subsequent steps of the method. Extracting features that from the point of view of the created model can contribute to increase its effectiveness, can occur as a result of testing various combinations of features. In this case, the significance of the features that affect the result in a given iteration is taken into account. Exemplary techniques and/or metrics to evaluate the effectiveness of models created based on various combinations of features are indicated and described later in this disclosure.

An exemplary record created for the described embodiment is presented below which also uses additional optional features. In addition, for a better understanding of the record, the middle column "feature description" contains explanation of particular parameters but this column is not part of the created record.

| Feature name | Feature description | Value |
| --- | --- | --- |
| AGE | Age | 46.00 |
| CH | Corneal hysteresis | 8.30 |
| CRF | Corneal resistance factor | 8.80 |
| tp2_wake_median_HR | Spearman's correlation coefficient for attribute values of TF and HR in subperiod tp2-wake | 0.00 |
| tp2_wake_median_DAP | Spearman's correlation coefficient for attribute values of TF and DAP in subperiod tp2-wake | 0.00 |
| start_tp1_median_MAP | Spearman's correlation coefficient for attribute values of TF and MAP in subperiod start-tp1 | 0.00 |
| sleep_tp2_median_SAP | Spearman's correlation coefficient for attribute values of TF and SAP in subperiod sleep-tp2 | 0.00 |
| tp3_end_median_CO | Spearman's correlation coefficient for attribute values of TF and CO in subperiod tp3-end | 0.00 |
| start_tp1_rawTf_sum | Area under the TF curve in subperiod start-tp1 | 88660.83 |
| tp1_sleep_rawTF_sum | Area under the TF curve in subperiod tp1-sleep | 77579.86 |
| sleep_tp2_rawTF_sum | Area under the TF curve in subperiod sleep-tp2 | 91247.26 |
| tp2_wake_rawTF_sum | Area under the TF curve in subperiod tp2-wake | 93400.43 |
| wake_tp3_rawTF_sum | Area under the TF curve in subperiod wake-tp3 | 190358.86 |
| tp3_end_rawTF_sum | Area under the TF curve in subperiod tp3-end | 160358.86 |
| start_tp1_SAP_sum | Area under the SAP curve in a given subperiod | 2313300.00 |
| tp1_sleep_SAP_sum | Area under the SAP curve in a given subperiod | 1990800.00 |
| sleep_tp2_SAP_sum | Area under the SAP curve in a given subperiod | 1049400.00 |
| tp2_wake_SAP_sum | Area under the SAP curve in a given subperiod | 1018500.00 |
| wake_tp3_SAP_sum | Area under the SAP curve in a given subperiod | 929400.00 |
| tp3_end_SAP_sum | Area under the SAP curve in a given subperiod | 2211300.00 |
| start_tp1_DAP_sum | Area under the DAP curve in a given subperiod | 1575000.00 |
| tp1_sleep_DAP_sum | Area under the DAP curve in a given subperiod | 1278900.00 |
| sleep _tp2_DAP_sum | Area under the DAP curve in a given subperiod | 574800.00 |

(continued)

| Feature name | Feature description | Value |
|---|---|---|
| tp2_wake_DAP_sum | Area under the DAP curve in a given subperiod | 587100.00 |
| wake_tp3_DAP_sum | Area under the DAP curve in a given subperiod | 528300.00 |
| tp3_end_DAP_sum | Area under the DAP curve in a given subperiod | 1323300.00 |
| start_tp1_MAP_sum | Area under the MAP curve in a given subperiod | 1811400.00 |
| tp1_sleep_MAP_sum | Area under the MAP curve in a given subperiod | 1510500.00 |
| sleep_tp2_MAP_sum | Area under the MAP curve in a given subperiod | 728400.00 |
| tp2_wake_MAP_sum | Area under the MAP curve in a given subperiod | 727500.00 |
| wake_tp3_MAP_sum | Area under the MAP curve in a given subperiod | 660300.00 |
| tp3_end_MAP_sum | Area under the MAP curve in a given subperiod | 1614000.00 |
| start_tp1_SAP_slope | Regression line slope of the SAP values in a given subperiod | -0.000786 |
| tp1_sleep_SAP_slope | Regression line slope of the SAP values in a given subperiod | 0.000615 |
| sleep_tp2_SAP_slope | Regression line slope of the SAP values in a given subperiod | 0.002841 |
| tp2_wake_SAP_slope | Regression line slope of the SAP values in a given subperiod | -0.002833 |
| wake_tp3_SAP_slope | Regression line slope of the SAP values in a given subperiod | 0.001666 |
| tp3_end_SAP_slope | Regression line slope of the SAP values in a given subperiod | -0.001515 |
| start_tp1_DAP_slope | Regression line slope of the DAP values in a given subperiod | -0.000146 |
| tp1_sleep_DAP_slope | Regression line slope of the DAP values in a given subperiod | 0.000175 |
| sleep_tp2_DAP_slope | Regression line slope of the DAP values in a given subperiod | 0.003079 |
| tp2_wake_DAP_slope | Regression line slope of the DAP values in a given subperiod | -0.001222 |
| wake_tp3_DAP_slope | Regression line slope of the DAP values in a given subperiod | 0.002444 |
| tp3_end_DAP_slope | Regression line slope of the DAP values in a given subperiod | -0.002286 |
| start_tp1_MAP_slope | Regression line slope of the MAP values in a given subperiod | -0.000362 |
| tp1_sleep_MAP_slope | Regression line slope of the MAP values in a given subperiod | 0.000315 |
| sleep_tp2_MAP_slope | Regression line slope of the MAP values in a given subperiod | 0.003063 |
| tp2_wake_MAP_slope | Regression line slope of the MAP values in a given subperiod | -0.001777 |
| wake_tp3_MAP_slope | Regression line slope of the MAP values in a given sub-period | 0.002222 |

(continued)

| Feature name | Feature description | Value |
|---|---|---|
| tp3_end_MAP_slope | Regression line slope of the MAP values in a given subperiod | -0.002029 |
| start_tp1_TF_sec_deriv_integral | Total variation of the scaled TF in a given subperiod | 0.004761 |
| tp1_sleep_TF_sec_deriv_integral | Total variation of the scaled TF in a given subperiod | 0.009872 |
| sleep_tp2_TF_sec_deriv_integral | Total variation of the scaled TF in a given subperiod | -0.003252 |
| tp2_wake_TF_sec_deriv_integral | Total variation of the scaled TF in a given subperiod | -0.006620 |
| wake_tp3_TF_sec_deriv_integral | Total variation of the scaled TF in a given subperiod | -0.040998 |
| tp3_end_TF_sec_deriv_integral | Total variation of the scaled TF in a given subperiod | 0.000116 |
| start_tp1_rawTF_sec_deriv_integral | Total variation of the original TF in a given subperiod | -0.002090 |
| tp1_sleep_rawTF_sec_deriv_integral | Total variation of the original TF in a given subperiod | 0.004761 |
| sleep_tp2_rawTF_sec_deriv_integral | Total variation of the original TF in a given subperiod | 0.009872 |
| tp2_wake_rawTF_sec_deriv_integral | Total variation of the original TF in a given subperiod | -0.003252 |
| wake_tp3_rawTF_sec_deriv_integral | Total variation of the original TF in a given subperiod | -0.006620 |
| tp3_end_rawTF_sec_deriv_integral | Total variation of the original TF in a given subperiod | -0.040998 |
| start_tp1_SAP_sec_deriv_integral | Total variation of the SAP values in a given subperiod | 0.010350 |
| tp1_sleep_SAP_sec_deriv_integral | Total variation of the SAP values in a given subperiod | 0.058333 |
| sleep_tp2_SAP_sec_deriv_integral | Total variation of the SAP values in a given subperiod | -0.052361 |
| tp2_wake_SAP_sec_deriv_integral | Total variation of the SAP values in a given subperiod | -0.044305 |
| wake_tp3_SAP_sec_deriv_integral | Total variation of the SAP values in a given subperiod | -0.094736 |
| tp3_end_SAP_sec_deriv_integral | Total variation of the SAP values in a given subperiod | -0.038518 |
| start_tp1_DAP_sec_deriv_integral | Total variation of the DAP values in a given subperiod | -0.057925 |
| tp1_sleep_DAP_sec_deriv_integral | Total variation of the DAP values in a given subperiod | -0.045490 |
| sleep_tp2_DAP_sec_deriv_integral | Total variation of the DAP values in a given subperiod | -0.019444 |
| tp2_wake_DAP_sec_deriv_integral | Total variation of the DAP values in a given subperiod | 0.042777 |
| wake_tp3_DAP_sec_deriv_integral | Total variation of the DAP values in a given subperiod | 0.030555 |
| tp3_end_DAP_sec_deriv_integral | Total variation of the DAP values in a given subperiod | -0.070175 |
| start_tp1_MAP_sec_deriv_integral | Total variation of the MAP values in a given subperiod | -0.057925 |
| tp1_sleep_MAP_sec_deriv_integral | Total variation of the MAP values in a given subperiod | -0,136470 |
| sleep_tp2_MAP_sec_deriv_integral | Total variation of the MAP values in a given subperiod | -0.007777 |
| tp2_wake_MAP_sec_deriv_integral | Total variation of the MAP values in a given subperiod | 0.042777 |
| wake_tp3_MAP_sec_deriv_integral | Total variation of the MAP values in a given subperiod | 0.012222 |
| tp3_end_MAP_sec_deriv_integral | Total variation of the MAP values in a given subperiod | -0.031578 |

In step s105 a label indicating a diagnosis made by a medical specialist (diseased/healthy) is assigned to each record (i.e. a set of features describing a single subject). As a result of gathering many such records created analogously for each of the many examined subjects a learning set is obtained which is used by the methods of supervised machine learning to construct a predictive model. In the described embodiment, the learning set was created on the basis of 120 records created for each of 120 subjects, who were examined and appropriately diagnosed by a medical specialist.

The predictive model is created in step **s106** using supervised machine learning mechanisms. The term supervised machine learning mechanisms used in this description should be understood as one or more algorithms, such as regression algorithms, e.g. Generalized Linear Model (GLM), decision tree algorithms), Bayesian algorithms e.g. Naive Bayes, ensemble algorithms e.g. Gradient Boosting Machine, support vector-based algorithms (SVM). It is not an exhaustive list and the skilled person in the art will also notice similar algorithms that can be equally used, although they are not specifically mentioned here. Details and rules of functioning of the mentioned algorithms have already been widely described in the literature, so for the sake of clarity they are not discussed in detail here. Examples of literature items:

- Pattern Recognition and Machine Learning; Christopher Bishop; Springer 2006
- Applied Predictive Modeling; Kjell Johnson, Max Kuhn; Springer 2013
- The Elements of Statistical Learning, 2nd edition; T.Hastie, R.Tibshirani; Springer 2008
- Python Machine Learning; S.Raschka, Packt Publishing 2015

In other words, the most important contribution of the present invention is the feature set which is determined in accordance with the principles described above and which constitutes the set of input data. Based on this input data, it is possible to create a predictive model using any appropriate supervised machine learning techniques, examples of which are indicated above. The selection of appropriate algorithms is therefore of secondary nature and can be carried out in many different ways and in various combinations obvious to those skilled in the art.

In the described embodiment, in order to create a predictive model, two algorithms from the above non-limiting list of supervised machine learning algorithms were selected and used: Generalized Linear Model (GLM) and Gradient Boosting Machine (GBM). Below, these algorithms are described in more detail in terms of their use in the described example, however, it should be noted that the invention is not limited to the indicated combination.

GLM (Generalized Linear Model) are generalized linear models with binomial distribution (equivalent to logistic regression). Models in this class use the properties of the logistic function to estimate the probability in binary classification. Below details of logistic regression are described that can be helpful for understanding the present invention.

For the binomial distribution (classification labels from the set {0,1}, where 0 - NORM) a probability model (a posteriori) P (C = c | X = x) is built with the following properties:

$$P(C = 0 \,|\, X=x) = \frac{\exp(\beta_0 + \beta^T X)}{1+\exp(\beta_0 + \beta^T x)}$$

and

$$P(C = 1 \,|\, X=x) = \frac{1}{1+\exp(\beta_0 + \beta^T x)}$$

The monotonic transformation in this case is a so-called logit: log (p/(1-p)). Accordingly:

$$\log\frac{P(C = 0|X=x)}{P(C = 1|X=x)} = \beta_0 + \beta^T x$$

The hyperplane separating classes is a set of points {x: $\beta_0 + \beta^T x = 0$}

The logistic regression model is fitted to the input data using the maximum likelihood method. Maximum reliability for N observations is given by the general formula

$$l(\theta)=\sum_{i=1}^{N} \log p_{g_i}(x_i \,;\, \theta),$$

where $p_k(x_i;\, \theta)=P(G = k|X=x;\, \theta)$

In the case of binomial distribution, in order to maximize l(β), zeros of derivative $\frac{\partial l(\beta)}{\partial \beta} = \sum_{i=1}^{N} x_i\big(y_i - p(x_i;\beta)\big) = 0$ (formula for binomial distribution - labels {0,1}) are found.

GBM (Gradient Boosting Machine) is a sequential committee/series of classifiers minimizing classification errors, with variable weight of committee components. This method uses a weighted average to calculate the final result, where the reduced dependence of the classifier components is obtained by random drawing subsets of training variables. GBM (Gradient Boosting Machine) algorithm is based on decision trees that divide the data space into separate square subsets. For each of these subsets, a dividing strategy is used that minimizes the mean square error (MSE) of the prediction $\sum_{i=1}^{N}(y_i - \overline{y_i})^2$ , where $\overline{y_i}$ is an estimated classification of the i-th observation. Subsequent decision trees $h_i$ are iteratively built, the algorithm result is the sum of trees from the generated string:

$$F_m(x) = F_{m-1}(x) + \gamma_m h_m(x)$$

where the coefficient $\gamma_m \in (0,1]$ is the weight of the generated tree (learning rate) added to the string / committee. The next decision tree in the m-th iteration is generated for the remainder obtained in the previous iteration $R(x) = f(x) - F_{m-1}(x)$. The error (represented by the loss function) is minimized by the method of the steepest gradient descent for the selected loss function (e.g. square error).

In step **s107,** classification models are created for the selected subset of attributes using 10-fold cross-validation to assess the accuracy of the prediction. Cross-validation (CV) is a model validation method that allows to assess the prediction error, and therefore allows to assess how effective the created model is. Accordingly, cross-validation can be used, for example, as a tool to compare models based on specific combinations of features and/or combinations of algorithms to identify the most advantageous configuration of the predictive model.

In the case of k-fold cross-validation, the input data set is randomly divided into k equal subsets. In iteration from 1 to k, another subset is selected that will be the test set. The remaining k - 1 subsets are combined and used as a training set to create the model. The generated results (for selected error measures) of cross validation (k numbers) are finally averaged (average or median) to estimate the prediction error of the model (with determined parameters and attributes).

The following metrics were used to evaluate binary models/classifiers:

- Accuracy: Accuracy = (TP + TN) / (number of all cases), where TP - true positive +, TN - true negative -

- logistic loss function (log loss)

- AUC (area under the ROC curve)

- mean square error (MSE)

In order to assess the variance / standard deviation of the cross-validation results, 50 iterations were performed for the procedure of calculating the above mentioned metrics.

AUC (Area Under the Curve) is the area under the ROC (Receiver Operating Characteristic) curve and it represents the accuracy of prediction based on sensitivity (TPR - true positive rate) and 1 - specificity (FPR - false positive rate). The classifier specificity is the TNR (true negative rate) equal to the ratio of the number of subsets of cases classified as negative from the set of negative cases). The ROC curve maps parametrically TPR(t) to FPR(t) for the variable parameter t, a so-called cut-off point. AUC of the ideal model is 1. The model providing the inversion of the reference classification has AUC equal to 0.

The logarithmic loss function (log loss) is a measure of the accuracy of prediction, which tends slowly to 0 for the model approaching the ideal pattern. In the case of incorrect predictions, the value of L increases.

$$L(y, \overline{y}) = -y \cdot log(\overline{y}) - (1 - y) \log(1 - \overline{y})$$

where prediction $\overline{y} \in [0,1]$. For the test set, the average of $L(y,\overline{y})$ of individual elements of this set is calculated.

MSE (Mean Squared Error) is a measure of the accuracy of model predictions that corresponds to the mean squared of deviations of the prediction from the correct value.

$$SE = \frac{1}{N}\sum_{i=1}^{N}(y_i - \overline{y_i})^2,$$

where $\overline{y_i}$ - estimated classification of the i-th observation, $y_i$ - correct classification of the i-th observation.

Examples of models created using GLM, GBM algorithms for various sets of attributes (versions 1 to 4) with their effectiveness measures are presented below:

|  | Attributes | AUC | MSE | Log loss |
|---|---|---|---|---|
| Version 1 | "CH", "CRF", "AGE", "tp2_wake_median_HR", "sleep-tp1_rawTF_sec_deriv_integral", "wake_tp3_rawTF_sum" | 0.89 ± 0.022 | 0.09493394 ± 0.016 | 1.792935 ± 0.4985968 |

(continued)

| | Attributes | AUC | MSE | Log loss |
|---|---|---|---|---|
| Version 2 | "CH", "CRF", "tp2_wake_median_HR", " sleep-tp1_rawTF_sec_deriv_integral ", "wake_tp3_rawTF_sum" | 0.88 ± 0.015 | 0.1311883 ± 0.011 | 0.5147769 ± 0.06530407 |
| Version 3 | "CH", "tp1_sleep_median_MAP", "AGE", "tp2_wake_median_HR", "sleep-tp1_rawTF_sec_deriv_integral", "wake_tp3_rawTF_sum" | 0.94 ± 0.017 | 0.095 ± 0.022 | 1.837 ± 0.528 |
| Version 4 | tp1_sleep_median_MAP", "tp2_wake_median_HR", "sleep-tp1_rawTF_sec_deriv_integral", "wake_tp3_rawTF_sum" | 0.91 ± 0.028 | 0.114 ± 0.019 | 0.618 ± 0.538 |

where CH is corneal hysteresis, CRF is corneal resistance factor, AGE is the subject's age, tp2_wake_median_HR is the Spearman's correlation coefficient for attribute values of TF and HR in subperiod TP2-WAKE, sleep-tp1_rawTF_sec_deriv_integral is the total variation of the SAP values in subperiod SLEEP-TP1, wake_tp3_rawTF_sum is the area under the curve of TF profile in subperiod WAKE-TP3, tp1_sleep_median_MAP is the correlation coefficient for coefficient parameters of TF and MAP in subperiod TP1-SLEEP.

The predictive model for which the construction method is described above is used to classify subjects by an inventive device. Similar to the construction of the predictive model, the data describing a subject being examined must be processed to obtain a set of corresponding features (the same features as the ones used in the model). Then the model is run on this data set and in consequence a result is generated in the form of allocation to one of the groups: diseased, healthy, along with the probability of this prediction.

The proposed solution can be used in an intelligent physician decision support system which will allow for an ultra-early assessment of the risk of developing glaucoma neuropathy and to describe its factors in the population of people observed for glaucoma, subjects with a positive family history and ocular hypertension. Furthermore, the system will enable personalization of local and systemic therapy in glaucomatous subjects, indicating individual risk factors for disease progression.

Fig. 7 shows a schematic block diagram of an exemplary device for implementing the method for creating a predictive model and the method for predicting glaucoma risk in a subject according to the invention. The device **200** comprises a control circuit **203** in which a processor **204** and a memory **205** are installed, the memory **205** being operatively coupled to the processor **204**. To the control circuit **203** means **201a** and means **201b** are coupled that are adapted to recording subject's eyeball parameters and cardiovascular system parameters, respectively. As the means **201a** and **201b** any suitable means can be used such as, e.g., the systems described above. These means record time profiles of parameters of the examined subject which are sent to the control circuit **203** via connections **202a** and **202b** and stored in the memory **205**. Furthermore, in the memory **205** a program code **206** is stored which, when executed by the processor **204,** causes the implementation of the subsequent steps of the above-described methods. The result of the processing performed by the processor in the form of classification of the examined subject as diseased or healthy along with determined probability is transmitted via connection **202c** to an output device **207** such as a display or monitor screen which presents the said result and other information related to the parameters and/or features determined from the profiles provided by the means **201a** and **201b** (such as correlation parameters, correlation heat maps etc. determined during creation of the predictive model) e.g. to a physician so that he can take them into account e.g. during diagnosis and select appropriate treatment. Optionally, an input device (not shown) may also be provided, such as a keyboard or pointing device, connected to the control circuit **203,** which will allow for the selection of the desired data to be presented by the output device **207.**

In a particular embodiment of the device **200** according to the invention, the means **201a** and **201b** and/or the output device **207** are arranged in a remote location relative to the control circuit **203.** In such case, the respective connections **202a, 202b** and/or **202c** are implemented as communication network connections, e.g. Internet network connections. This provides greater flexibility in the use of the device **200** according to the invention. For example, in one possible scenario, a subject uses means **201a** and **201b** for recording the described parameters which during recording are collected in a local memory of the means **201a** and **201b,** and then, once the data collection is completed, the collected data is sent automatically or by a person helping to carry out the examination, for example via a dedicated network connection, to the control circuit **203** implemented e.g. in the form of a server. Once the data is processed by the control circuit **203,** the result can be sent back to the output device **207,** e.g. for presentation to a person carrying out the examination (e.g. a physician).

Although the invention has been described in detail with reference to the specific embodiments set out above, this description is by way of example and is not intended to limit the invention to specific embodiments. The skilled person will appreciate that various changes and modifications are possible without departing from the scope of the invention as defined by the following claims.

**Claims**

1. A method **(100)** for creating a predictive model for predicting glaucoma risk in a subject, the method comprising:

   a step of creating a diagnostic model comprising, for each one of a plurality of subjects:

   a) recording **(s101a)** a 24-hour profile of eyeball parameters and simultaneously recording **(s101b)** cardiovascular system parameters;
   b) dividing **(s102a)** the recorded 24-hour profile of eyeball parameters at least into subperiods:

   - an initial subperiod **START** - **TP1** from session start to 5 hours before assuming a horizontal position for sleep;
   - a subperiod preceding assuming a horizontal position for sleep **TP1- SLEEP** from 5 hours before assuming a horizontal position to assuming a horizontal position for sleep;
   - a subperiod following assuming a horizontal position for sleep **SLEEP - TP2** from assuming a horizontal position for sleep to assuming a horizontal position for sleep + 2 hours;
   - a subperiod preceding assuming a vertical position after sleep **TP2** - **WAKE** from assuming a horizontal position for sleep + 2 hours to assuming a vertical position after sleep;
   - a subperiod following assuming a vertical position after sleep **WAKE = TP3** from assuming a vertical position after sleep to assuming a vertical position after sleep + 2 hours;
   - a final subperiod **TP3-END** from assuming a vertical position after sleep + 2 hours to the session end;

   c) determining **(s103a),** in each subperiod, features describing a single subject in the form of at least one aggregating attribute and calculating **(s103b)** correlations between the eyeball parameters and the cardiovascular system parameters;
   d) creating **(s104)** a record containing the determined features describing a single subject and the calculated correlation parameters;
   e) assigning **(s105)** a label indicating a diagnosis (diseased/healthy) made by a physician to the created record; and

   a step of creating a predictive model, based on a set of records created for the plurality of subjects, using supervised machine learning mechanisms based on one or more algorithms selected at least from regression algorithms, decision trees, Bayesian algorithms, ensemble algorithms and support vector-based algorithms, the predictive model receiving a record of a patient to be examined, the record containing the same feature set as the one created in step d) **(s104)** and outputting the probability of said record to belong to a group of healthy subjects or to a group of diseased subjects.

2. The method according to claim 1, wherein the predictive model is created using 10-fold cross-validation.

3. The method according to claim 1 or 2, wherein the aggregating attributes are selected from a group including: a sum of the area under the curve in a subperiod, the slope angle of a linear regression line in a subperiod, the total variation in a subperiod, representative values of the discrete Fourier transform in a subperiod.

4. The method according to any one of the preceding claims, wherein the eyeball parameters are selected from a group including: the circumference at the corneoscleral limbus of an eyeball and intraocular pressure.

5. The method according to any one of the preceding claims, wherein the cardiovascular system parameters are selected from a group including: blood pressure (BP): systolic arterial pressure (SAP), diastolic arterial pressure (DAP), mean arterial pressure (MAP), heart rate (HR), oxygen blood saturation (SpO2) and cardiac output fraction calculated according to the formula: $CO = [(SAP-DAP)/SAP + DAP] \times HR$.

6. The method according to any one of the preceding claims, wherein one or more additional features selected from a group including: subject's age, corneal resistance factor and corneal hysteresis are determined **(s103c)** and appended to the record describing a single subject created in the step **(s104)** of the method **(100).**

7. The method according to any one of the preceding claims, wherein the record describing a single subject in the step **(s104)** of the method **(100)** is limited to a selected subset of the all determined features.

8. The method according to any one of the preceding claims, wherein the determined subperiods furthermore include a subperiod from the session start to assuming a horizontal position for sleep **(START - SLEEP)** and/or a subperiod from assuming a horizontal position for sleep to assuming a vertical position after sleep **(SLEEP -WAKE)** and/or a subperiod from assuming a horizontal position at 14:00 to assuming a vertical position at 15:30 with sustained consciousness **(TIME 14:00 - TIME 15:30).**

9. A method for determining glaucoma risk in a subject, the method comprising:

   - creating, for a patient to be examined, a record containing the same feature set as the one created in the step **(s104)** of the method **(100)** for creating a predictive model according to any one of claims 1-8,
   - determining the subject's feature similarity to a group of diseased or healthy subjects with determined probability using the predictive model created according to the method of any one of claims 1-8.

10. A device for predicting glaucoma in a subject, comprising:

    means **(201a)** for recording eyeball parameters;
    means **(201b)** for recording cardiovascular system parameters;
    a control circuit **(203)** having a communication connection **(202a, 202b)** with both the means for recording **(201a, 201b),** and comprising:

    a processor **(204);**
    a memory **(205)** operatively coupled to the processor **(204),** containing instructions that, when executed by the processor, implement the method according to any one of claims 1-9 based on data provided by the means **(201a, 201b)** for recording; and
    an output device **(207)** for presenting results having a communication connection **(202c)** with the control circuit **(203).**

11. The device according to claim 10, wherein
    the means **(201a)** for recording eyeball parameters, the means **(201b)** for recording cardiovascular system parameters and/or the output device **(207)** are arranged in a remote location with respect to the control circuit **(203),** and the communication connections **(202a, 202b, 202c)** are communication network connections.

12. A computer program comprising a program code that, when executed by a processor, causes the processor to perform the steps of the method as defined in claims 1-8 and of the method as defined in claim 9.

13. A computer readable medium on which the computer program of claim 12 is stored.

**Patentansprüche**

1. Verfahren **(100)** zum Erstellen eines Vorhersagemodells zur Vorhersage des Glaukomrisikos bei einem Patienten, wobei

   das Verfahren:

   einen Schritt zum Erstellen eines Diagnosemodells, das für jedes einer Vielzahl von Patienten:

   a) Aufzeichnen **(s101a)** eines 24-Stunden-Profils von Augapfelparametern und gleichzeitiges Aufzeichnen **(s101b)** von Parametern des kardiovaskulären Systems;
   b) Aufteilen **(s102a)** des aufgezeichneten 24-Stunden-Profils von Augapfelparametern in mindestens Teilzeiträumen:

   - ein anfänglicher Teilzeitraum **START - TP1** vom Sitzungsbeginn bis 5 Stunden vor der Einnahme einer horizontalen Position zum Schlafen;

- ein Teilzeitraum vor der Einnahme einer horizontalen Position zum Schlafen **TP1 - SLEEP** von 5 Stunden vor dem Einnahme einer horizontalen Position bis zur Einnahme einer horizontalen Position zum Schlafen;

- ein Teilzeitraum nach der Einnahme einer horizontalen Position zum Schlafen **SLEEP - TP2** von der Einnahme einer horizontalen Position zum Schlafen bis zur Einnahme einer horizontalen Position zum Schlafen + 2 Stunden;

- ein Teilzeitraum vor der Einnahme einer vertikalen Position nach dem Schlafen **TP2 - WAKE** von der Einnahme einer horizontalen Position zum Schlafen + 2 Stunden bis zur Einnahme einer vertikalen Position nach dem Schlafen;

- ein Teilzeitraum nach der Einnahme einer vertikalen Position nach dem Schlafen **WAKE - TP3** von der Einnahme einer vertikalen Position nach dem Schlafen bis zur Einnahme einer vertikalen Position nach dem Schlafen + 2 Stunden;

- ein letzter Teilzeitraum TP3- von der Einnahme einer vertikalen Position nach dem Schlafen + 2 Stunden bis zum Sitzungsende;

c) Bestimmen **(s103a)** in jedem Teilzeitraum von Merkmalen, die einen einzelnen Patienten in Form von mindestens einem aggregierenden Attribut beschreiben, und Berechnen **(s103b)** von Korrelationen zwischen den Augapfelparametern und den Parametern des kardiovaskulären Systems;

d) Erstellen **(s104)** eines Datensatzes, der die bestimmten Merkmale, die einen einzelnen Patienten beschreiben, und die berechneten Korrelationsparameter enthält:

e) Zuweisen **(s105)** einer Bezeichnung, die eine von einem Arzt gestellte Diagnose (krank/gesund) angibt, zu dem erstellten Datensatz;

umfasst, und

einen Schritt zum Erstellen eines Vorhersagemodells auf der Grundlage eines Satzes von Datensätzen, die für die Vielzahl von Patienten erstellt wurden, unter Verwendung überwachter maschineller Lernmechanismen auf der Grundlage eines oder mehrerer Algorithmen, die zumindest aus Regressionsalgorithmen, Entscheidungsbäumen, Bayes-Algorithmen, Ensemble-Algorithmen und Support-Vektor-basierten Algorithmen ausgewählt wurden,

wobei das Vorhersagemodell einen Datensatz eines zu untersuchenden Patienten empfängt, wobei der Datensatz denselben Merkmalssatz enthält wie der in Schritt d) **(s104)** erstellte Datensatz, und die Wahrscheinlichkeit ausgibt, dass dieser Datensatz zu einer Gruppe gesunder Patienten oder zu einer Gruppe erkrankter Patienten gehört,

umfasst.

2. Verfahren nach Anspruch 1, wobei das Vorhersagemodell unter Verwendung einer 10-fachen Kreuzvalidierung erstellt wird.

3. Verfahren nach Anspruch 1 oder 2, wobei die aggregierenden Attribute aus einer Gruppe ausgewählt werden, die eine Summe der Fläche unter der Kurve in einem Teilzeitraum, den Steigungswinkel einer linearen Regressionslinie in einem Teilzeitraum, die Gesamtvariation in einem Teilzeitraum, repräsentative Werte der diskreten Fourier-Transformation in einem Teilzeitraum, umfasst.

4. Verfahren nach einem der vorhergehenden Ansprüche, wobei die Augapfelparameter aus einer Gruppe ausgewählt werden, die den Umfang am korneoskleralen Limbus eines Augapfels und den Augeninnendruck umfasst.

5. Verfahren nach einem der vorhergehenden Ansprüche, wobei die Parameter des kardiovaskulären Systems aus einer Gruppe ausgewählt werden, die Blutdruck (BP): systolischer arterieller Druck (SAP), diastolischer arterieller Druck (DAP), mittlerer arterieller Druck (MAP), Herzfrequenz (HR), Sauerstoffsättigung im Blut (SpO2) und Herzleistungsanteil, berechnet nach der Formel: $CO = [(SAP-DAP)/SAP + DAP)] \times HR$, umfasst.

6. Verfahren nach einem der vorhergehenden Ansprüche, wobei ein oder mehrere zusätzliche Merkmale, ausgewählt aus einer Gruppe, die

Alter des Patienten, Hornhautwiderstandsfaktor und Hornhauthysterese, bestimmt **(s103c)** und an den Datensatz angehängt werden, der einen einzelnen Patienten beschreibt und im Schritt **(s104)** des Verfahrens **(100)** erstellt wurde, umfasst.

7. Verfahren nach einem der vorhergehenden Ansprüche, wobei der Datensatz, der einen einzelnen Patienten im Schritt **(s104)** des Verfahrens **(100)** beschreibt, auf eine ausgewählte Teilmenge aller ermittelten Merkmale beschränkt ist.

8. Verfahren nach einem der vorhergehenden Ansprüche, wobei die ermittelten Teilzeiträume außerdem einen Teilzeitraum vom Sitzungsbeginn bis zur Einnahme einer horizontalen Position zum Schlafen **(START** - **SLEEP)** und/oder einen Teilzeitraum von der Einnahme einer horizontalen Position zum Schlafen bis zur Einnahme einer vertikalen Position nach dem Schlafen **(SLEEP** - **WAKE)** und/oder einen Teilzeitraum von der Einnahme einer horizontalen Position um 14:00 bis zur Einnahme einer vertikalen Position um 15:30 bei anhaltendem Bewusstsein **(TIME 14:00** - **TIME 15:30)** umfassen.

9. Verfahren zur Bestimmung des Glaukomrisikos bei einem Subjekt,

   wobei das Verfahren:

   - Erstellen eines Datensatzes für einen zu untersuchenden Patienten, der denselben Merkmalssatz enthält wie der, der im Schritt **(s104)** des Verfahrens **(100)** zum Erstellen eines Vorhersagemodells gemäß einem der Ansprüche 1-8 erstellt wurde, und,
   - Bestimmen der Merkmalsähnlichkeit des Patienten mit einer Gruppe von kranken oder gesunden Patienten mit bestimmter Wahrscheinlichkeit unter Verwendung des gemäß dem Verfahren eines der Ansprüche 1-8 erstellten Vorhersagemodells,

   umfasst.

10. Gerät zur Vorhersage eines Glaukoms bei einem Patienten, umfassend:

    Mittel **(201a)** zum Aufzeichnen von Augapfelparametern;
    Mittel **(201b)** zum Aufzeichnen von Parametern des kardiovaskulären Systems;
    eine Steuerschaltung **(203),** die eine Kommunikationsverbindung **(202a, 202b)** mit beiden Mitteln zum Aufzeichnen **(201a, 201b)** aufweist und

    einen Prozessor **(204)**;
    einen Speicher **(205),** der operativ mit dem Prozessor **(204)** verbunden ist und Anweisungen enthält, die, wenn sie vom Prozessor ausgeführt werden, das Verfahren gemäß einem der Ansprüche 1 bis 9 auf der Grundlage von Daten implementieren, die von den Mitteln **(201a, 201b)** zum Aufzeichnen bereitgestellt werden; und
    ein Ausgabegerät **(207)** zum Präsentieren von Ergebnissen, das eine Kommunikationsverbindung **(202c)** mit der Steuerschaltung **(203)** aufweist, umfasst.

11. Das Gerät gemäß Anspruch 10, wobei die Mittel **(201a)** zum Aufzeichnen von Augapfelparametern, die Mittel **(201b)** zum Aufzeichnen von Parametern des kardiovaskulären Systems und/oder das Ausgabegerät **(207)** an einem entfernten Ort in Bezug auf die Steuerschaltung **(203)** angeordnet sind und die Kommunikationsverbindungen **(202a, 202b, 202c)** Kommunikationsnetzwerkverbindungen sind.

12. Ein Computerprogramm, das einen Programmcode umfasst, der, wenn er von einem Prozessor ausgeführt wird, den Prozessor dazu veranlasst, die Schritte des in den Ansprüchen 1-8 definierten Verfahrens und des in Anspruch 9 definierten Verfahrens auszuführen.

13. Ein computerlesbares Medium, auf dem das Computerprogramm von Anspruch 12 gespeichert ist.

**Revendications**

1. Procédé **(100)** de création d'un modèle prédictif pour prédire le risque de glaucome chez un patient, le procédé comprenant:

   une étape de création d'un modèle de diagnostic comprenant, pour chacun d'une pluralité de patients:

   a) enregistrement **(s101a)** d'un profil de 24 heures de paramètres du globe oculaire et enregistrement simultané **(s101b)** de paramètres du système cardiovasculaire;
   b) division **(s102a)** du profil de 24 heures enregistré de paramètres du globe oculaire au moins en sous-périodes:

   - une sous-période initiale **START - TP1** du début de la session à 5 heures avant la prise une position horizontale pour le sommeil;
   - une sous-période précédant la prise d'une position horizontale pour le sommeil **TP1**
   - **SLEEP** de 5 heures avant la prise d'une position horizontale à la prise d'une position horizontale pour le sommeil;
   - une sous-période suivant la prise d'une position horizontale pour le sommeil **SLEEP**
   - **TP2** de la prise d'une position horizontale pour le sommeil à la prise d'une position horizontale pour le sommeil + 2 heures;
   - une sous-période précédant la prise d'une position verticale après le sommeil **TP2 - WAKE** de la prise d'une position horizontale pour le sommeil + 2 heures à la prise d'une position verticale après le sommeil;
   - une sous-période suivant la prise d'une position verticale après le sommeil **WAKE** - **TP3** de la prise d'une position verticale après le sommeil à la prise d'une position verticale après le sommeil + 2 heures;
   - une sous-période finale **TP3-END** de la prise d'une position verticale après le sommeil + 2 heures à la fin de la session;

   c) déterminer **(s103a),** dans chaque sous-période, des caractéristiques décrivant un seul patient sous la forme d'au moins un attribut d'agrégation et calculer **(s103b)** des corrélations entre les paramètres du globe oculaire et les paramètres du système cardiovasculaire;
   d) créer **(s104)** un enregistrement contenant les caractéristiques déterminées décrivant un seul patient et les paramètres de corrélation calculés;
   e) attribuer **(s105)** une étiquette indiquant un diagnostic (maladie/sain) posé par un médecin sur l'enregistrement créé; et

   une étape de création d'un modèle prédictif, basé sur un ensemble sur l'enregistrement créés pour la pluralité des patients, en utilisant des mécanismes d'apprentissage automatique supervisés basés sur un ou plusieurs algorithmes sélectionnés au moins parmi des algorithmes de régression, des arbres de décision, des algorithmes bayésiens, des algorithmes d'ensemble et des algorithmes basés sur des vecteurs de support,
   le modèle prédictif recevant un enregistrement d'un patient à examiner, l'enregistrement contenant le même ensemble de caractéristiques que celui créé à l'étape d) **(s104)** et délivrant en sortie la probabilité que ledit enregistrement appartienne à un groupe de patients sains ou à un groupe de patients malades.

2. Procédé selon la revendication 1, dans lequel le modèle prédictif est créé en utilisant une validation croisée 10 fois.

3. Procédé selon la revendication 1 ou 2, dans lequel les attributs d'agrégation sont sélectionnés dans un groupe comprenant: une somme de l'aire sous la courbe dans une sous-période, l'angle de pente d'une ligne de régression linéaire dans une sous-période, la variation totale dans une sous-période, des valeurs représentatives de la transformée de Fourier discrète dans une sous-période.

4. Procédé selon l'une quelconque des revendications précédentes, dans lequel les paramètres du globe oculaire sont sélectionnés dans un groupe comprenant: la circonférence au niveau du limbe cornéoscléral d'un globe oculaire et la pression intraoculaire.

5. Procédé selon l'une quelconque des revendications précédentes, dans lequel les paramètres du système cardiovasculaire sont sélectionnés dans un groupe comprenant: pression artérielle (PA); pression artérielle systolique (PAS), pression artérielle diastolique (PAD), pression artérielle moyenne (PAM), fréquence cardiaque (FC), saturation en oxygène du sang (SpO2) et fraction de débit cardiaque calculée selon la formule : CO = [(PAS-PAD)/PAS + PAD)] x FC.

6. Procédé selon l'une quelconque des revendications précédentes, dans lequel une ou plusieurs caractéristiques supplémentaires sélectionnées dans un groupe comprenant: l'âge du patient, le facteur de résistance cornéenne et l'hystérésis cornéenne sont déterminées **(s103c)** et ajoutées à l'enregistrement décrivant un seul patient créé à l'étape **(s104)** du procédé **(100).**

7. Procédé selon l'une quelconque des revendications précédentes, dans lequel l'enregistrement décrivant un seul patient à l'étape **(s104)** du procédé **(100)** est limité à un sous-ensemble sélectionné de toutes les caractéristiques déterminées.

8. Procédé selon l'une quelconque des revendications précédentes, dans lequel les sous-périodes déterminées comprennent en outre une sous-période allant du début de la session à la prise d'une position horizontale pour le sommeil **(START - SLEEP)** et/ou une sous-période allant de la prise d'une position horizontale pour le sommeil à la prise d'une position verticale après le sommeil **(SLEEP- WAKE)** et/ou une sous-période allant de la prise d'une position horizontale à 14h00 à la prise d'une position verticale à 15h30 avec une conscience soutenue **(TIME 14:00 - TIME 15:30).**

9. Procédé de détermination du risque de glaucome chez un patient, le procédé comprenant:

   - création, pour un patient à examiner, d'un enregistrement contenant le même ensemble de caractéristiques que celui créé à l'étape **(s104)** du procédé **(100)** de création d'un modèle prédictif selon l'une quelconque des revendications 1 à 8,
   - détermination de la similarité des caractéristiques du patient avec un groupe de patients malades ou sains avec une probabilité déterminée en utilisant le modèle prédictif créé selon le procédé selon l'une quelconque des revendications 1 à 8.

10. Dispositif de prédiction du glaucome chez un patient, comprenant:

    des moyens **(201a)** pour enregistrer des paramètres du globe oculaire;
    des moyens **(201b)** pour enregistrer des paramètres du système cardiovasculaire;
    un circuit de commande **(203)** ayant une connexion de communication **(202a, 202b)** avec les deux moyens pour enregistrement **(201a, 201b),** et comprenant:

    un processeur **(204);**
    une mémoire **(205)** couplée de manière opérationnelle au processeur **(204),** contenant des instructions qui, lorsqu'elles sont exécutées par le processeur, mettent en œuvre le procédé selon l'une quelconque des revendications 1 à 9 sur la base de données fournies par les moyens **(201a, 201b)** d'enregistrement; et
    un dispositif de sortie **(207)** pour présenter des résultats ayant une connexion de communication **(202c)** avec le circuit de commande **(203).**

11. Dispositif selon la revendication 10, dans lequel
    les moyens (201a) pour enregistrer des paramètres du globe oculaire, les moyens **(201b)** pour enregistrer des paramètres du système cardiovasculaire et/ou le dispositif de sortie **(207)** sont disposés dans un emplacement distant par rapport au circuit de commande **(203),** et les connexions de communication **(202a, 202b, 202c)** sont des connexions de réseau de communication.

12. Programme informatique comprenant un code de programme qui, lorsqu'il est exécuté par un processeur, amène le processeur à exécuter les étapes du procédé tel que défini dans les revendications 1 à 8 et du procédé tel que défini dans la revendication 9.

13. Support lisible par ordinateur sur lequel le programme informatique selon la revendication 12 est stocké.

**Method 100 for creating a predictive model**

Fig. 1

Fig. 2

Fig. 3

Fig. 4

START      14:00 15:00 .TP1       .SLEEP   .TP2            .WAKE .TP3      .END

Fig. 5

EP 3 893 248 B1

**■■ Applanation signal ■■ Pressure (air pulse)**

Fig. 6

Fig. 7

26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **WASILEWICZ RH** ; **WASILEWICZ PK** ; **MAZUREK C et al.** Daily biorhythms of ocular volume changes and the cardiovascular system functional parameters in healthy, ocular hypertension, normal tension and primary open angle glaucoma populations. *ARVO Annual Meeting Abstract*, April 2014
- Use of Machine Learning on Contact Lens Sensor-Derived Parameters for the Diagnosis of Primary Open-angle Glaucoma. **MARTIN KEITH R et al.** American Journal of Ophthalmology. Elsevier, 25 July 2018, vol. 194, 46-53
- Towards glaucoma detection using intraocular pressure monitoring. **GISLER CHRISTOPHE et al.** 2014 6th International Conference of Soft Computing and Pattern Recognition (SoCPaR). IEEE, 11 August 2014, 255-260
- **CHRISTOPHER BISHOP**. Pattern Recognition and Machine Learning. Springer, 2006
- **KJELL JOHNSON** ; **MAX KUHN**. Applied Predictive Modeling. Springer, 2013
- **T.HASTIE** ; **R.TIBSHIRANI**. The Elements of Statistical Learning. Springer, 2008
- **S.RASCHKA**. Python Machine Learning. Packt Publishing, 2015